# EUROPÄISCHE PATENTSCHRIFT

(11) **EP 3 307 868 B1**
(45) Veröffentlichungstag und Bekanntmachung des Hinweises auf die Patenterteilung: **01.04.2020**
(21) Anmeldenummer: 16729236.6
(22) Anmeldetag: 08.06.2016
(51) Int. Cl.: C12M 1/42, C12M 3/04

(54) **MODULARES BIOREAKTORSYSTEM**
MODULAR BIOREACTOR SYSTEM
SYSTÈME DE BIORÉACTEUR MODULAIRE

(30) Priorität: 10.06.2015 DE 102015210609
(43) Veröffentlichungstag der Anmeldung: 18.04.2018
(73) Patentinhaber: Ospin GmbH, 10827 Berlin (DE)
(72) Erfinder: SAAM, Jan, 10965 Berlin (DE); ERFURTH, Hendrik, 12623 Berlin (DE); HANSMANN, Jan, 97286 Sommerhausen (DE); SCHRÖDER, Dimitrij, 70569 Stuttgart (DE); SCHWARZ, Thomas, 97286 Sommerhausen (DE); KRZIMINSKI, Steffan, 97080 Würzburg (DE)
(74) Vertreter: Schwahn, Hartmut
(86) Internationale Anmeldenummer: PCT/EP2016/062970
(87) Internationale Veröffentlichungsnummer: WO 2016/198423

(56) Entgegenhaltungen:
- WO-A1-2010/115185
- WO-A2-2012/170878
- DE-A1-102008 015 633
- US-A1- 2003 143 727

## Beschreibung

Die vorliegende Erfindung betrifft ein modulares Bioreaktorsystem für Tissue Engineering-Anwendungen, besonders zur pulsatilen unidirektionalen Perfusion biologischer Zellen und Gewebe sowie eine Pumpanordnung und Pumpe.

Die Funktion von Bioreaktoren bei der Züchtung künstlicher Gewebe und Organe ist Bereitstellung gewebetypischer physiologischer Bedingungen, die den Zellen eine natürliche Umgebung simulieren, so, wie sie in dem jeweiligen Gewebe vorzufinden ist. Dazu gehören neben der Einstellung von Temperatur und pH auch die Versorgung mit Nährstoffen, der Abtransport von Stoffwechselprodukten und das Errichten einer gewebetypischen Reizkulisse. Zu den Reizen zählen v.a. mechanische, elektrische und biochemische Stimulation. Bestimmte medizinische und biotechnologische Anwendungen erfordern eine realistische Simulation physiologischer Druck- und Flussbedingungen. Der durch vorbeiströmende Flüssigkeit hervorgerufene Scherstress, sowie die durch pulsierende Druckverhältnisse erzeugte periodische Dehnung sind wichtige morphogenetische und Differenzierungsreize für die Zellen in kardiovaskulären Geweben. Im sogenannten Tissue Engineering entstehen bei der Kultivierung von Arterien oder Herzklappen im Perfusionsbioreaktor nur dann funktionelle Gewebemodelle, wenn sie pulsatil perfundiert werden. In bekannten Anwendungen dieses Bereichs kommen zumeist Schlauchpumpen zum Einsatz deren Rotationsgeschwindigkeit insbesondere elektronisch so geregelt ist, dass ein sich periodisch ändernder Volumenstrom im System entsteht.

Bei sehr kleinen Gewebemodellen z.B. bei Hanging-Drop Kulturen und bei speziellen Gewebetypen werden, abhängig von Fragestellung und Verwendungszweck, bereits bei statischer Kultivierung zufriedenstellende Ergebnisse erzielt. Statische Kultur heißt, dass die Gewebemodelle im Reaktionsgefäß nicht dynamisch vom Kulturmedium umspült oder durchspült werden, sondern von ruhendem Medium umgeben sind. Eine dynamische Kultivierung hingegen hilft, die Versorgung größerer Gewebestücke sicherzustellen und für die entsprechende physiologische mechanische Reizkulisse zu sorgen. Die mechanische Stimulation besonders durch Dehnung, Druck oder Scherkräfte stellen wichtige Aktivierungs- und Differenzierungsreize dar und/oder bieten Orientierung bei der räumlichen Selbstorganisation der biologischen Zellen. Eine Arterie besteht im Wesentlichen aus der Endothelschicht, einer Schicht tangential orientierter glatter Muskelzellen und einer Bindegewebsschicht mit Fibroblasten. Wird eine poröse Gerüststruktur (scaffold) mit diesen Zelltypen besiedelt, so sorgt erst die mechanische Stimulation in der dynamischen Kultivierung für die natürliche Positionierung und Orientierung der Zellen. Endothelzellen haben "Sensoren" für die Scherkräfte der durch die Perfusion vorbeiströmenden Medienflüssigkeit. Sie erkennen daran die physiologische "innere Oberfläche" eines Gefäßes und kleiden sie deshalb mit einer konfluenten Schicht vollständig aus. Glattmuskelzellen hingegen wandern in tiefere Schichten ein und reagieren auf Dehnungsreize die besonders durch eine Perfusion mit pulsierendem Druck (pulsatile Perfusion) ausgelöst wird mit Ausrichtung in tangentialer Richtung. Erst diese Orientierung ermöglicht im Gefäß die Fähigkeit zur Verengung (Vasokonstriktion) und Erweiterung (Vasodilatation) des Gefäßdurchmessers. Ähnliche Mechanismen lassen sich beim Tissue Engineering vieler anderer Gewebe, etwa bei Epithelien wie Darm, Lunge oder mehrschichtiger Haut nutzen. Auch bei der Herstellung vaskularisierter Gewebe werden pulsatile Perfusionssysteme eingesetzt. Perfusionsbioreaktoren mit pulsatiler Perfusion sind also ein wichtiges Werkzeug bei der Herstellung künstlicher Gewebe.

Für Gewebeproduktion im klinischen Umfeld müssen außerdem bereits etablierte Herstellungsprotokolle die Sicherheitsstandards genügen und dokumentiert sind, eingehalten werden, wodurch der Einsatz speziell für den jeweiligen Anwendungskontext konzipierter Bioreaktorsysteme sinnvoll ist. Für Tissue Engineering Anwendungen im Forschungsstadium aber ist Flexibilität im Aufbau eines Bioreaktorsystems von großer Bedeutung. Der Aufbau der Bioreaktorsysteme ist anwendungsabhängig; bekannte Komplettsysteme, die spezifisch für klinischen Aufgaben konzipiert sind, müssten dazu aber aufwändig herstellerseitig rekonfiguriert werden.

In Forschungslaboren finden sich häufig Systeme, die sich für Temperatur- und pH-Kontrolle (über CO2-Austausch) auf so genannte Zellkulturinkubatoren stützen. Dabei müssen die Pumpen zur gewünschten pulsatilen Perfusion entweder außerhalb oder innerhalb des Inkubators eingesetzt werden. Eine solche Anordnung ist einerseits sehr raumintensiv und andererseits komplex im Aufbau und nicht gut zugänglich und Tissue Engineering-Anwendungen mit solchen Systemen, die zwar für den Forschungseinsatz tauglich sind, implizieren bekanntermaßen viel manuelle Arbeit, besonders beim Setup, Medienwechsel, Monitoring, Reinigen, etc. Zudem stellen solche Systeme meist komplexe Geräte oder Geräteverbünde dar, die durch Anwender schwer oder aufwändig zu bedienen und zu warten sind.

US 2003/143727 offenbart eine Anordnung zur Kultivierung von Zellkonstrukten zur Gewebeherstellung unter physiologisch-ähnlichen Bedingungen, wie z.B. zur pulsatilen Druckbeaufschlagung während des Kultivierungsprozesses, damit durch den Stimulus widerstandfähige Zellkonstrukte entstehen, wie z.B. Blutgefäßkonstrukte.

Es wird ein daher ein Bioreaktorsystem gefordert, das flexibel in der Anwendung und skalierbar zugleich ist. Es soll ermöglicht werden, parallele Tests parallel durchzuführen. Dies ist erforderlich für mehr Vergleichsdaten und bessere Statistik in der Forschung. Außerdem soll die Reproduzierbarkeit der Experimente verbessert werden, da sonst nur begrenzte Vergleichbarkeit der Ergebnisse besteht.

In solchen bekannten Perfusionsbioreaktorsystemen, aber auch losgelöst von der Aufgabe der dynamischen Kultivierung in anderen biotechnologischen und medizinischen Anwendungen z.B. Dialysesysteme müssen Flüssigkeiten wie Zellkulturmedium oder Blut gefördert werden. Zur Vermeidung von Kontaminationen, müssen dabei alle fluid-/medienführenden Bestandteile des Systems vor der Verwendung sterilisiert werden. Die fluidführenden Teile bilden idealerweise ein geschlossenes System das als Ganzes vorsterilisiert und bis zum Einsatz gelagert werden kann, das so genannte Fluidiksystem.

Es sollen Einwegsysteme zum Einsatz kommen, bei denen der Fluidikteil von dem Rest der Anlage konstruktiv getrennt ist und nach einmaligem Gebrauch entsorgt werden kann. Solche Einwegsysteme sichern die Sterilität und Kontaminationsfreiheit oder ersparen dem Anwender die aufwändige Reinigung. Sie bestehen aus kostengünstigen Massen-Materialien wie Kunststoffschläuchen, Plastikbeuteln, Kunststoffkonnektoren usw. Ein typischer Bestandteil von zum Einsatz solcher Fluidiksysteme geeigneter Bioreaktoren sind peristaltische Pumpen insbesondere so genannte Schlauchpumpen, bei denen der auswechselbare medienführende Teil durch einen flexiblen Schlauch dargestellt wird.

Nachteilig bei bekannten Pumpsystemen, welche auf peristaltischen Pumpen basieren, ist unter anderem ein geringes Pumpvolumen, was dicke Perfusionsschläuche erforderlich macht. Eventuell im gepumpten Medium flotierende Zellen werden durch die Mechanik der peristaltischen Pumpen permanent mechanischem Stress ausgesetzt, was zur ungewollten Aktivierung oder gar Zerstörung dieser Zellen führen kann. Der elektrische Antrieb bekannter peristaltischer Pumpen ist ab einer gewissen Pumprate neben einer hohen Geräuschemission mit einer Wärmeentwicklung verbunden, die insbesondere bei kompakten Aufbauten nachteilig ist und einer zuverlässigen und flexiblen Temperaturregulierung des Systems entgegensteht. Durch die Anordnung des Elektromotors sind außerdem flache Geometrien schwer zu realisieren. Ebenfalls ist mit peristaltischen Pumpen die Drucksteuerung im System schwierig, da die Drücke in der Regel innerhalb des Fluidiksystems gemessen werden müssen. Besonders gibt es für Einwegdrucksensoren für Fluidiksysteme nur kurze Standzeiten. Nichtinvasive Sensoren sind sehr teuer.

Alternative Ansätze zur pulsatilen Perfusion beruhen daher auf dem Prinzip einer Membranpumpe oder Kolbenpumpe. Eine Aufteilung bekannter Pumpsystem in eine Hauptkammer und eine Vorkammer hat den Nachteil, dass in der aktiven Pumpphase, der sogenannten Diastole, kein Medium im Kreislauf transportiert sondern lediglich die Hauptkammer aus einer Vorkammer gefüllt wird. Das sogenannte "Glätten" des pulsierenden Flusses, wie es in der Peripherie des Körpers, im Abstand vom Herzen oder den Schlagadern natürlicherweise vorkommt und für die meisten Anwendungen bei der pulsatilen Perfusion von Gewebe zweckmäßig wäre, ist dabei nur mit verhältnismäßig großen Windkesseln (compliance chamber) möglich.

Ein Nachteil solcher bekannter Perfusionssysteme und Pumpen für die pulsatile Perfusion ist auch, dass an sich bekannte sterilisierbare Perfusionsbestecke, wie beispielsweise Blutbeutel oder Infusionsbeutel mit konfektionierten Schlauchsystemen, die als Einwegartikel ohne weiteres erhältlich sind, nicht eingesetzt werden können.

Daneben besteht die Forderung nach einer guten Skalierbarkeit von Bioreaktorsystemen. Diese ist begründet einerseits durch die Notwendigkeit, in der Forschung eine ausreichende Anzahl an parallelen Versuchen und Kontrollen für eine gute statistische Fundierung der Forschungsergebnisse durchzuführen. Andererseits fokussiert die Mehrzahl der Tissue Engineering-Forschungsprojekte auf konkrete klinische, diagnostische oder pharmazeutische Anwendungen. Voraussetzung für diese Anwendungen ist, dass es möglich wird, Gewebe in größerer Stückzahl und in reproduzierbarer Qualität herzustellen. Bekannte skalierbare Systeme für den klinischen und industriellen Einsatz fokussieren jeweils auf ganz bestimmte Gewebe bzw. Anwendungen und können durch den Anwender kaum umkonfiguriert werden. Es fehlen Systeme, die eine Skalierung der Gewebeherstellung im Rahmen forschungsrelevanter Stückzahlen erlauben ohne die im Forschungsbereich wichtige Flexibilität und Konfigurierbarkeit aufzugeben. Weiterhin fehlen Ansätze, die die Lücke zwischen dem Forschungsbereich und der industriellen Produktion überspannen, vor allem Systeme, die in der Produktentwicklung die Skalierung eines Tissue Engineering Prozesses in kleinen Schritten ohne Technologiebruch ermöglichen.

Vor diesem Hintergrund bestand das der Erfindung zugrunde liegende technische Problem in der Bereitstellung eines verbesserten Perfusionsbioreaktorsystems mit verbesserter Pumpe zur pulsatilen Perfusion, besonders für Tissue Engineering-Anwendungen, insbesondere zur Perfusion von biologischen Zellen oder Geweben. Es soll zum Einen eine pulsatile Perfusion der Zellen oder Gewebe in einer Gewebekammer unter realistischer Stimulation physiologischer Druck- und Flussbedingungen ermöglicht werden, zum anderen soll dabei auf einfach sterilisierbare Perfusionsbestecke zurückgegriffen und zur Perfusion eingesetzt werden können.

Weiter bestand das technische Problem, ein Bioreaktorsystem bereitzustellen, das es erlaubt, flexibel an das jeweilige Gewebemodell und die Test- oder Kultivierungsbedingungen angepasst zu werden. Dabei sollen in einer Gewebekammer perfundierte Zellen oder Gewebe während der Perfusion auch für zeitweise Untersuchungen oder für ein dauerhaftes Monitoring verfügbar sein, beispielsweise eine mikroskopische Beobachtung der Zellen oder Gewebe. Daneben soll eine Skalierung des Gesamtsystems einfach und reproduzierbar an das jeweilige Gewebemodell ermöglicht werden. In jedem Fall sollen dabei im Wesentlichen dieselben Grundkomponenten verwendbar sein.

Die zugrunde liegenden technischen Probleme werden in einem ersten Aspekt der Erfindung vollständig gelöst durch die Bereitstellung einer Pumpanordnung, welche geeignet ist und insbesondere speziell ausgebildet ist, zur unidirektionalen, insbesondere pulsatilen Perfusion von biologischen Zellen oder Gewebe in einer separaten Gewebekammer, welche mit der Pumpanordnung in Fluidverbindung gebracht werden kann. Erfindungsgemäß weist die Pumpanordnung eine erste flexible Flüssigkeitskammer und zumindest eine zweite flexible Flüssigkeitskammer auf. Beide Flüssigkeitskammern sind insbesondere in Form von bevorzugt flachen Folienbeuteln ausgebildet. Erfindungsgemäß ist der ersten flexiblen Flüssigkeitskammer ein erstes passives Einlassventil zugeordnet, das mit dieser insbesondere über eine eigene Leitung in Fluidverbindung steht. Weiter ist der ersten flexiblen Flüssigkeitskammer ein erstes passives Auslassventil zugeordnet, das mit dieser insbesondere über eine eigene Leitung in Fluidverbindung steht. Erfindungsgemäß ist der zweiten flexiblen Flüssigkeitskammer ein zweites passives Einlassventil zugeordnet, das mit dieser insbesondere über eine eigene Leitung in Fluidverbindung steht. Weiter ist der zweiten flexiblen Flüssigkeitskammer ein zweites passives Auslassventil zugeordnet, das mit dieser insbesondere über eine eigene Leitung in Fluidverbindung steht. Erfindungsgemäß sind die Eingänge des ersten und zweiten Einlassventils gemeinsam über einen Einlassport verbunden und stehen mit diesem in Fluidverbindung. Weiter sind die Ausgänge des ersten und zweiten Auslassventils mit einem Auslassport verbunden und stehen mit diesem in Fluidverbindung. Die Flussrichtung zur unidirektionalen Perfusion läuft von Einlassport über die beiden Einlassventile und die beiden flexiblen Flüssigkeitskammern zu den beiden Auslassventilen und zu dem Auslassport.

Die erfindungsgemäße Pumpanordnung gemäß Anspruch 1 zeichnet sich also vor allem durch zwei über passive Ventile gegenläufig verschaltete Pumpkammern in Form flexibler Flüssigkeitsbeutel aus, welche zur Durchführung der unidirektionalen Perfusion von Flüssigkeiten, insbesondere biologischer Medien, jeweils alternierend durch mindestens einen externen Aktor druckbeaufschlagbar sind. Dazu sieht die Erfindung bevorzugt vor, dass in der ersten Flüssigkeitskammer ein erster Verdrängungsaktor zugeordnet ist, der geeignet ist, die erste Flüssigkeitskammer insbesondere wiederkehrend mit Druck zu beaufschlagen; der zweiten Flüssigkeitskammer ist ein zweiter Verdrängungsaktor zugeordnet, der mit dieser in mechanischer Wirkverbindung steht und geeignet ist, die zweite Flüssigkeitskammer, insbesondere wiederkehrend, und alternierend zur ersten mit Druck zu beaufschlagen, um im Zusammenspiel einen pulsatilen Nettofluss durch die Pumpanordnung zu bewirken.

Zur Verwirklichung der wiederkehrenden alternierenden Druckbeaufschlagung von erster und zweiter Flüssigkeitskammer ist bevorzugt jede Flüssigkeitskammer zusammen mit dem jeweiligen ihr zugeordneten Verdrängungsaktor in einem starren, in Bezug auf die Druckbeaufschlagung mechanisch druckfesten Gehäuse unterbringbar und bevorzugt untergebracht.

Demgemäß ist der Gegenstand der Erfindung eine Pumpanordnung, welche ein erstes starres Gehäuseteil zur Aufnahme der ersten Flüssigkeitskammer mit einem ersten Verdrängungsaktor aufweist, zur Druckbeaufschlagung der ersten Flüssigkeitskammer und welche ein zweites starres Gehäuseteil zur Aufnahme der zweiten Flüssigkeitskammer aufweist mit einem zweiten Verdrängungsaktor zur Druckbeaufschlagung der zweiten Flüssigkeitskammer. Bevorzugt sind das erste und zweite starre Gehäuseteil in einem integralen Gesamtgehäuse untergebracht. Besonders sind die Gehäuseteile zusammen als integrale Kammer ausgebildet, wobei insbesondere die beiden Gehäuseteile jeweils separate Abschnitte in der integralen geschlossenen Kammer bilden.

Die erfindungsgemäße Pumpanordnung sieht insbesondere vor, als erste und zweite flexible Flüssigkeitskammern Folienbeutel, besonders flache Folienbeutel, einzusetzen, insbesondere aber sterilisierbare Einweg-Folienbeutel. Für die Funktion ist die Art der verwendeten Beutel im Prinzip unerheblich, solange sie eine flexible Wand besitzen und den mechanischen Belastungen standhalten können. Diese sind bevorzugt ausgewählt aus: Blutbeuteln, Infusionsbeuteln, Kryobeuteln, Zellkulturbeuteln und ähnlichen Systemen. In der Praxis sind medizinische Infusionsbeutel oder Zellkulturbeutel ausreichend stabile, kostengünstige und vor allem anerkannt biokompatible Varianten. Da mindestens ein Einlass und mindestens ein Auslass benötigt wird, sind konfektionierte Infusionsbeutel mit zwei oder zwei paarigen oder mehr Anschlüssen (ports) bevorzugt. In bevorzugter Ausgestaltung sind die Folienbeutel in an sich bekannter Weise aus zwei Folienblättern zusammengeschweißt. Bevorzugte Folienmaterialien sind EVA, LDPE, PET, PP, soft-PP, PVC, PU, PTFE, PA, PA6, Verbundfolien daraus oder vergleichbare Materialien, besonders bevorzugt ist EVA- oder PP-Verbundfolie. In einer bevorzugten Variante ist die eingesetzte Folie des Folienbeutels gasdurchlässig, insbesondere CO2-durchlässig. In einer alternativen Ausgestaltung ist die Folie im Wesentlichen gasdicht.

Die flüssigkeitsführenden Teile eines Bioreaktors, das so genannte Fluidiksystem, ist bevorzugt als geschlossenes vorsterilisiertes System, insbesondere als Einwegsystem ausgeführt, das als Ganzes in das Gesamtsystem einlegbar ist. Die flexiblen Flüssigkeitskammern der erfindungsgemäßen Pumpe sind damit Bestandteil dieses Fluidiksystems. Zu diesem Zwecke sind die Gehäuseteile der erfindungsgemäßen Pumpe bevorzugt so ausgeführt, dass die flexiblen Flüssigkeitskammern, insbesondere in der Ausgestaltung als flache Folienbeutel, jeweils von einer Seite durch schlitzförmige Öffnungen in die Gehäuseteile einsetzbar oder einschiebbar sind. Die erfindungsgemäßen Einlass- und Auslassleitungen, und insbesondere auch die Einlass- und Auslassventile, befinden sich in dieser Ausgestaltung jeweils auf einer einzigen Seite der Flüssigkeitskammern, bevorzugt auf der kurzen Seite eines rechteckigen Folienbeutels.

Erfindungsgemäß bevorzugt ist also das starre Gehäuse im Wesentlichen vollständig geschlossen aber weist lediglich, und bevorzugt ausschließlich, eine seitliche Öffnung, bevorzugt in Form eines Schlitzes auf. Diese Öffnung ist zur temporären Aufnahme der Flüssigkeitskammern ausgebildet. Bevorzugt ist jedem Gehäuseteil eine eigene schlitzförmige Öffnung zugeordnet, worin dezidiert die für diesen Gehäuseteil bestimmte flexible Flüssigkeitskammer zum Betrieb der Pumpe aufgenommen werden kann.

Zusätzlich können die Gehäuseteile mit einer Vorrichtung, besonders Klemme, Verriegelung, etc., versehen werden, die das Herausrutschen der Flüssigkeitskammern oder Beutel während des Betriebs verhindert. Zusätzlich oder alternativ ist die schlitzförmige Öffnung zur Aufnahme eines flachen Folienbeutels ausgebildet und so ausgestaltet und dimensioniert, dass die Flüssigkeitskammern in der Phase der Druckbeaufschlagung durch den jeweiligen Verdrängungsaktor (Systole) durch die Reib- und Klemmkräfte des Verdrängungsaktors und der Gehäusewand in dem Gehäuse gehalten werden und in der Druckentlastungsphase (Diastole) durch deren dann größere Dicke aufgrund der Füllung nicht durch die schmalere schlitzförmige Öffnung treten können und so in dem Gehäuse gehalten werden.

Bevorzugt ist der Verdrängungsaktor in dem jeweiligen Gehäuseteil ein flexibler, insbesondere über eine pneumatische Zuleitung druckbeaufschlagbarer Luftbeutel, der in dem starren Gehäuseteil temporär oder dauerhaft aufgenommen ist. In einer alternativen Ausgestaltung ist der Verdrängungsaktor als druckdichte Luftkammer in dem jeweiligen Gehäuseteil selbst ausgebildet, wobei die Luftkammer über eine insbesondere flexible Membran oder eine verschiebliche Trennwand mit der einsetzbaren Flüssigkeitskammer zu deren Druckbeaufschlagung in Wirkverbindung kommen kann. Anstatt eines speziellen Balgs oder einer Kammer mit flexibler Membran kann für die Luft einfach der gleiche Beutel wie für die Flüssigkeit verwendet werden, der dann ebenfalls in das Standgehäuse eingesetzt ist.

Bevorzugt werden zumindest einer oder beide Aktoren mit einem oder mehreren Drucksteuerventilen in Verbindung. Diese Ventile sind geeignet, den jeweiligen Verdrängungsaktor druckgesteuert und/oder zeitgesteuert mit Druck zu beaufschlagen. In einer bevorzugten Variante steht erster und zweiter Verdrängungsaktor jeweils mit einem ersten und zweiten aktiven Drucksteuerventil in druckfester Verbindung zum Zwecke der alternierenden druckgesteuerten Druckbeaufschlagung der Verdrängungsaktoren. Bevorzugt ist das Drucksteuerventil ein aktives Drucksteuerventil, besonders bevorzugt ein Proportionaldruckregelventil. In einem alternativen Ansatz wird die wiederkehrende Druckbeaufschlagung mindestens eines Verdrängungsaktors mithilfe von Wege- beziehungsweise Sperrventilen realisiert, welche insbesondere frequenzgesteuert sind. Dabei ist besonders vorgesehen, dass die Schlagfrequenz der Wege- beziehungsweise Sperrventile über die Taktung der Ventile eingestellt wird. In einer alternativen Variante davon ist vorgesehen, Wegebeziehungsweise Sperrventile mit Drucksteuerung einzusetzen. Es wird ein oberer und ein unterer Druck definiert, wobei umgeschaltet wird, sobald der jeweilige Druck erreicht ist. In einer bevorzugten Ausgestaltung sind die Einlassventile und Auslassventile jeweils über eine gemeinsame Zuleitung mit den ihnen zugeordneten Flüssigkeitskammern in Fluidverbindung. In einer alternativen Ausgestaltung sind Einlassventil und Auslassventil jeweils separat über eine eigene Einlass- beziehungsweise Auslasszuleitung mit der jeweiligen Flüssigkeitskammer verbunden.

In Flussrichtung nach der Pumpe kann eine Druckausgleichskammer (Windkessel, compliance chamber) in den Flüssigkeitsstrom geschaltet sein. Damit lässt sich der Strom "glätten" und somit ein kontinuierlicherer Fluss erzeugen. Im Vergleich zu einem herzähnlichen Aufbau mit in Reihe geschalteter Vor- und Hauptkammer kommt man hier mit einer sehr viel kleineren Ausgleichskammer aus, da nur die kurze Umschaltphase aber nicht die komplette diastolische Phase überbrückt werden muss. Mit dem Druck in der Ausgleichskammer lässt sich also die Federkonstante des Dämpfungsglieds einstellen - je höher der Druck desto größer die Federkonstante und desto härter die Dämpfung.

Für Zellkultur und Tissue Engineering müssen die Bereiche, welche die Zellen oder Gewebe enthalten, temperiert werden. Erfindungsgemäß bevorzugt sind die Flüssigkeitskammern oder Beutel in der Pumpkammer auch für den Wärmeaustausch und damit für die Temperierung des gesamten Systems, Fluidiksystem und anschließbarer Gewebekammer, vorgesehen. Es besteht dort vorteilhafterweise nur eine dünne Folie zwischen innerer Flüssigkeit und Außenseite bei vergleichsweise großer Austauschfläche.

In einer ersten Ausgestaltung ist in das Gehäuse ein Wärmetauscher, besonders in Form einer temperierten Platte integriert, die mit mindestens einer der Flüssigkeitskammern in unmittelbarem Wärmeflusskontakt steht. Alternativ oder zusätzlich ist das gesamte Gehäuse über einen Wärmetauscher beheizt oder gekühlt.

Im Rahmen dieser Erfindung und ihrer Beschreibung wird unter einem "Wärmetauscher" ein Wärme zu- und/oder abführendes Element (Heizelement, Kühlelement) verstanden. Die Erfindung sieht vor, dass der oder die Wärmetauscher die aufgenommenen Flüssigkeitskammern, und damit die in den Flüssigkeitskammern befindlichen flüssigen Medien temperieren. Dies kann durch positiven Wärmefluss (Heizung) aber gegebenenfalls auch durch negativen Wärmefluss (Kühlung) geschehen. Bevorzugt ist als Wärmetauscher ein elektrisches Heizelement vorgesehen. Alternativ weist der Wärmetauscher einen eigenen Medienkreislauf auf und ist mit flüssigem oder gasförmigem Medium betrieben, welches mit einer Wärmequelle und/oder einer Wärmepumpe zur Wärmeübertragung in Verbindung steht. In einer alternativen Ausgestaltung ist der Wärmetauscher ein Halbleiterheiz- und/oder -kühlelement (Peltier-Element) oder ähnliches.

Das Gehäuse oder wesentliche Teile davon sind dazu aus wärmeleitfähigem Material, insbesondere Aluminium, Magnesium oder diese enthaltenden Legierungen, aufgebaut. Das erfindungsgemäße direkte Beheizen des Systems über die Flüssigkeitskammern in der Pumpanordnung ermöglicht einen schnelleren Wärmeübertrag in das Fluidiksystem. Vor allem kann so die Temperierung des Systems eng geführt und kontrolliert werden. Wärmeverluste, wie sie beispielsweise bei längeren Zu- und Ableitungen in dem Fluidiksystem zwischen den einzelnen Elementen oder Modulen des Gesamtsystems, besonders zwischen Pumpe und Gewebekammer oder Gewebekammermodul, auftreten können, sind so durch die direkte Wärmekopplung innerhalb der Pumpanordnung leicht kompensierbar.

In bevorzugter Ausgestaltung ist zumindest ein Gehäuseteil, bevorzugt beide Gehäuseteile, bevorzugt die integrale Kammer, welche beide Gehäuseteile beherbergt, mit zumindest einem solchen Wärmetauscher ausgestattet. Bevorzugt ist je Gehäuseteil ein Wärmetauscher vorgesehen. In einer Variante davon ist ein gemeinsamer Wärmetauscher für beide Gehäuseteile vorgesehen. Erfindungsgemäß ist besonders vorgesehen, dass der Wärmetauscher Bestandteil des Gehäuseteils ist und insbesondere in dem Gehäuseteil integriert oder unmittelbar an dem Gehäuseteil angebracht ist. Bevorzugt sind Mittel und Maßnahmen vorgesehen, um die Wärmeübertragung zwischen Gehäuseteil und Wärmetauscher und insbesondere zwischen Wärmetauscher und in das Gehäuseteil jeweils aufgenommener Flüssigkeitskammer zu gewährleisten. Bevorzugt ist vorgesehen, dass der oder die Wärmetauscher an oder in dem Gehäuseteil so dimensioniert sind, dass durch die erfindungsgemäße Pumpanordnung in Verbindung mit dem oder den Wärmetauschern ausschließlich die Temperierung des flüssigen Mediums in einem Bioreaktorsystem übernimmt. Auf eine separate Heizung oder Wärmetauscheranordnung außerhalb des Pumpensystems wird vorzugsweise verzichtet. In dieser bevorzugten Ausführung eines Bioreaktors dient ausschließlich die erfindungsgemäße Pumpanordnung mit Wärmetauscher als die einzige Temperiereinheit in dem Bioreaktor.

Gegenstand eines zweiten Aspekts der Erfindung ist ein modulares Perfusions-Bioreaktorsystem, insbesondere für Tissue Engineering-Anwendungen zur Züchtung künstlicher Gewebe oder Organe unter gewebetypischen physiologischen Bedingungen, insbesondere zur pulsatilen Perfusion biologischer Zellen oder Gewebe. Bestandteil dieses modularen Bioreaktorsystems ist erfindungsgemäß zumindest ein generisches Hauptmodul für ein damit verbindbares separates anwendungsspezifisches Gewebekammermodul.

Die modulare Bioreaktorplattform sieht eine systematische Trennung applikationsspezifischer und generischer Komponenten vor. Bis auf die Gewebekammer sind alle oben beschriebenen Komponenten generisch und somit für eine große Anzahl von Anwendungen relevant. Diese sind in einem integralen Hauptmodul vereint und untergebracht. Applikationsspezifisch hingegen ist die Gewebekammer zusammen mit der das Gewebe betreffenden Sensorik und etwaigen Aktuatoren. Das gesamte Bioreaktorsystem folgt also einem modularen Aufbau, indem der Austausch eines Gewebekammermoduls ermöglicht ist, während die generischen Elemente in einem separaten Hauptmodul konstant verbleiben. Das Hauptmodul enthält diese genannten Funktionskomponenten, bevorzugt in einem einzigen Gehäuse integriert. Die aktiven Funktionskomponenten des Hauptmoduls sind über einen Rechner softwaregesteuert und in ihrer körperlichen Ausgestaltung konstant und generisch. Durch eine spezifische Programmierung oder eine spezifische Auswahl einer Programmvariante in dem Betriebssystem des Hauptmoduls kann dieses an das anschließbare anwendungsspezifische, d.h. besonders gewebespezifische, Gewebemodul variabel angepasst werden.

Eine Schnittstelle sorgt für die Kommunikation zwischen Hauptmodul und Gewebekammermodul. Verschiedene Gewebekammermodule haben bevorzugt jeweils eine digitale Signatur, die der Steuersoftware des Hauptmoduls eine automatische Erkennung ermöglicht. Eine modell- oder gewebespezifische Gewebekammer ist bevorzugt eindeutig identifizierbar nummeriert oder markiert und kann dazu vorzugsweise mit einem Barcode markiert werden, der von einem Scanner, der bevorzugt in dem Gewebekammermodul selbst angeordnet ist, erkannt werden. Das Softwaresystem kann diese Information um einen anwendungsspezifischen Satz von Konfigurationsparametern für einen anwendungsspezifischen Herstellungs- und Kultivierungsprozess aktivieren.

Gegenstand der Erfindung ist somit auch ein modulares Bioreaktorsystem, enthaltend ein generisches Hauptmodul und ein anwendungsspezifisches Gewebekammermodul zur Aufnahme von biologischen Zellen oder Gewebe, wobei das Gewebekammermodul von dem Hauptmodul räumlich getrennt und austauschbar ist.

Das Hauptmodul enthält zumindest eine hierin beschriebene erfindungsgemäße Pumpe, sowie außerdem ein Fluidiksystem zur Fluidverbindung der Pumpe mit einem dem Gewebekammermodul, weiter ein Drucksteuersystem zur Steuerung der Druckbeaufschlagung des ersten und/oder zweiten Verdrängungsaktors der Pumpe sowie zumindest eine Temperiereinheit mit Wärmetauscher.

Bevorzugt weist das Druckluftsteuersystem des Hauptmoduls mindestens ein hierin beschriebenes Drucksteuerventil auf, das mit mindestens einem der Aktoren in druckfester Verbindung steht, sowie einen Rechner, der mit dem mindestens einen Drucksteuerventil verbunden ist, zu dessen programmgemäßen Ansteuerung, besonders zur Steuerung des pulsatilen Flusses der Perfusion, insbesondere der Drucksteuerung in der Perfusion. Dazu ist in dem Hauptmodul mindestens ein mit diesem Rechner verbundener Drucksensor eingebaut zur direkten oder indirekten Messung des aktuellen Drucks in der Perfusion des Systems.

Das Hauptmodul enthält außerdem bevorzugt einen Gasaustauscher insbesondere in Form eines gasdurchlässigen Schlauchs, der in einer Austauschkammer verläuft, in der eine definierte Atmosphäre eingestellt werden kann, einen Blasenfänger, und ein gekühltes Kompartiment für darin gelagertes Frischmedium.

Zusätzlich kann das Hauptmodul eine weitere Pumpe für Perifusion (Umspülung) von Gewebe enthalten. Die parallele Perfusion (Durchspülung) und Perifusion (Umspülung) ist vor allem für zweiseitige, polare Gewebe oder mehrschichtige Gewebemodelle vorgesehen. Hier können sich Perfusionsmedium und Perifusionsmedium in Art und Zusammensetzung unterscheiden, um optimale Kultivierungsbedingungen für diese Gewebe zu schaffen. Vom Hauptkreislauf zweigt also optional ein Nebenkreislauf ab, der von einer eigenen Pumpe versorgt wird. Dieser Nebenkreislauf dient der optionalen Perifusion, also Umspülung, des Gewebemodells. In einer Variante sind Pumpe für Perfusion und Pumpe für Perifusion jeweils in der erfindungsgemäßen Art ausgebildet, wie hierin beschrieben. In einer Variante ist die Pumpe für Perifusion eine einfachere und nicht pulsatile Pumpe; die physiologischen pulsatilen Druckverhältnisse am Gewebe werden dabei bevorzugt ausschließlich durch die erfindungsgemäß ausgebildete Perfusion erzielt, durch geeignete Maßnahmen in dem geschlossenen System kann eine physiologisch zweckmäßige dynamische Druckdifferenz zwischen der an sich fließkonstanten Perifusion und der pulsatilen Perfusion erreicht werden, um das Gewebe adäquat mechanisch zu reizen.

Das zum Einsatz mit dem Hauptmodul vorgesehene Gewebekammermodul weist zumindest zwei geeignete Anschlüsse (Einlass, Auslass) für den Perfusionskreislauf als solchen auf. Eine Variante des Gewebekammermoduls weist zumindest zwei Kompartimente auf, ein Perfusionskompartiment mit zumindest zwei geeigneten Anschlüssen (Einlass, Auslass) für den Perfusionskreislauf und ein Perifusionskompartiment mit zumindest zwei geeigneten Anschlüssen (Einlass, Auslass) für den Perifusionskreislauf. Das zu kultivierende Gewebe wird zwischen die beiden Kompartimente eingesetzt und bildet das Interface zwischen diesen.

Alle Funktionskomponenten sind so auf bzw. in einer Grundplatte befestigt, dass von der Anwenderseite das Fluidiksystem eingelegt werden kann, während die Versorgung der Komponenten mit Strom, Druckluft und Messleitungen auf der vom Nutzer abgewandten Seite geschieht. Die Grundplatte ist in ein Gehäuse eingelassen und trennt dort ein in dem Hauptmodul integriertes Elektronik- und Steuerkompartiment, enthaltend insbesondere einen Rechner sowie Sensorik und Leistungselektronik, von einem anwenderzugänglichen Fluidik-Kompartiment. Das Fluidik-Kompartiment ist mit einem thermisch isolierten Deckel schließbar.

Der pH-Wert im Medium ist bevorzugt über die CO2-Konzentration einstellbar (für Bikarbonat-gepufferte Medien). Dazu wird Medium durch einen gasdurchlässigen Schlauch gepumpt, der durch eine Austauschkammer läuft in der die gewünschte CO2 Atmosphäre durch Mischung von Luft und CO2 eingestellt wird. Bevorzugt ist der CO2-Gasaustausch innerhalb der Gehäuseteile der Pumpanordnung vorgesehen. Beispielsweise kann CO2 als Betriebsgas des Verdrängungsaktors, der mit dem als Folienbeutel ausgeführten Flüssigkeitskammern in intimer Verbindung steht, eingesetzt werden. Auf eine separate Gastauscheranordnung außerhalb des Pumpensystems wird vorzugsweise verzichtet. In dieser bevorzugten Ausführung eines Bioreaktors dient ausschließlich die erfindungsgemäße Pumpanordnung als der einzige Gasaustauscher in dem Bioreaktor.

Wie vorstehend beschrieben, ist der Fluidpfad in dem Hauptmodul auch oder bevorzugt ausschließlich über die erfindungsgemäße Pumpanordnung mit Wärmetauscher direkt beheizt. Zusammen mit einem bevorzugt in die Gehäusewand der Pumpe eingelassenen Temperatursensor ist die Temperatur dort mess- und regelbar. In einer alternativen Ausgestaltung sind auch weitere Funktionskomponenten des Hauptmoduls temperiert, insbesondere eine separate Gasaustauscher-Komponente. In einer Variante davon ist die Grundplatte des Hauptmoduls, das die Funktionskomponenten trägt, insgesamt temperiert

Eine bevorzugte Ausführung des Bioreaktormoduls enthält eine Automatische Prozessteuerung, und insbesondere einen automatischen Medienwechsel, bevorzugt mit konstanter Rate oder alternativ basierend auf einer laufenden Messung von Nährstoffen und/oder Stoffwechselprodukten im System, z.B. Glukose oder Laktat.

Eine bevorzugte Ausführung des Bioreaktors betrifft eine generell Web-basierte Konfiguration. Der Rechner des Hauptmoduls fungiert dabei als Server und/oder als Client für Datenübertragung und Programmsteuerung in Verbindung mit damit verbindbaren Remote-Systemen.

Die Gewebekammer enthält bevorzugt einen Zellträger oder Gerüst (scaffold), was die Struktur und Form des zu kultivierenden Gewebes vorgibt; bevorzugt ist z.B. eine Röhre für ein Blutgefäßmodell-Gewebe). Der Zellträger wird vor der Sterilisation in die Kammer eingesetzt. Durch die Gewebekammer und den eingesetzten Zellträger wird das System anwendungsspezifisch.

In einer besonderen Ausführung ist das Gewebekammermodul derart ausgebildet, dass eine laufende, insbesondere langsame, Drehung der Gewebes im Schwerefeld während des Kultivierungsprozesses erfolgen kann, um beispielsweise eine gleichmäßige Besiedelung einer Gerüststruktur (scaffold) zu erzielen oder einen nachteiligen Einfluss der Schwerkraft zu eliminieren. Alternativ kann das Gewebekammermodul derart ausgebildet sein, dass durch anhaltende, insbesondere schnelle, Drehung der Gewebes ein zusätzliches Schwerefeld während des Kultivierungsprozesses erzeugt werden kann, um beispielsweise eine Schwerkrafttrennung oder Sedimentation der kultivierten Zellen oder Gewebe zu erzielen. Dazu kann das Gewebemodul, das ja erfindungsgemäß von dem Hauptmodul räumlich getrennt angeordnet sein kann, in eine separate Rotationseinheit eingesetzt werden, ohne dass diese nachteilig mit dem Bioreaktorsystem in Wechselwirkung kommt, und insbesondere kein Wärmeeintrag erfolgt.

Das Fluidiksystem des modularen Bioreaktorsystems, das Gewebekammermodul mit dem Hauptmodul verbindet, stellt ein geschlossenes System dar, das nach der Sterilisation gelagert werden kann. Das System bleibt geschlossen bis auf die Momente in denen das Medienreservoir angeschlossen bzw. gewechselt wird oder die Zellen in das System injiziert werden. Diese Konnektierungen können mit Hilfe spezieller Verbinder hergestellt werden, die die Kontaminationswahrscheinlichkeit reduzieren. Das Zellkulturmedium kann als zusätzliche Schutzmaßnahme über einen Sterilfilter mit dem Rest des Systems verbunden werden. Es ist besonders vorgesehen, dass der Flüssigkeitspfad als geschlossenes integrales Kreislaufsystem bestehend aus Beuteln, Schläuchen, Konnektoren, Blasenfänger und Gewebekammer als Ganzes in den Bioreaktor eingelegt werden kann. Der Flüssigkeitspfad ist bevorzugt als Single Use Fluid-Pfad ausgeführt, was Reinigung erspart und das Kontaminationsrisiko vermindert.

Der modulare Aufbau des erfindungsgemäßen Bioreaktorsystems, besonders die Aufteilung in einen konstanten, generischen Teil, nämlich das Hauptmodul, und einen davon trennbaren variablen, spezifischen Teil, nämlich das Gewebekammermodul mit der Gewebekammer, erlaubt nicht nur eine flexible und anwendungsspezifische Zusammenstellung der Module. Die erfindungsgemäß vorgesehene räumliche Trennung der Module ist mit entsprechend angepasstem Fluidiksystem auch während des Kultivierungsbetriebs möglich. Die Gewebekammer ist dazu in dem Gewebekammermodul bevorzugt so in einem Halter angebracht, so dass es möglich ist, die Gewebekammer im laufenden Betrieb herauszunehmen, ohne die Integrität und Sterilität des Flüssigkeitssystems zu stören. Das Flüssigkeitssystem verfügt dazu über ausreichend lange Verbindungsschläuche zur Gewebekammer, sodass die Gewebekammer in einem geeigneten externen Untersuchungsgerät, insbesondere ein Mikroskop oder Spektrometer, platziert werden kann. In einer Variante kann das gesamte Gewebekammermodul in die Untersuchungsmaßnahmen aufgenommen werden. Für den Zugang zu der Gewebekammer braucht dank der Erfindung nur das Gewebekammermodul selbst, nicht aber das Hauptmodul geöffnet werden, wodurch der Wärmeverlust und Störungen im Betriebsablauf minimiert, und die Betriebssicherheit erhöht werden

Gegenstand der Erfindung ist somit auch ein modulares Bioreaktorsystem, welches das vorstehend beschriebene Hauptmodul sowie mindestens ein Gewebekammermodul zur Aufnahme der biologischen Zellen oder Gewebe enthält, wobei das Gewebemodul während des Betriebes/der Kultivierung räumlich von dem Hauptmodul trennbar, insbesondere ab- oder herausnehmbar, ohne dass der Perfusionskreislauf unterbrochen werden oder der Kultivierungsbetrieb eingestellt werden muss. Die Erfindung betrifft somit auch ein Perfusionsbioreaktorsystem zur flexiblen automatischen Kultivierung von Zellen oder Gewebe mit integrierter Inkubatorfunktion und modular austauschbarer Gewebekammer.

Die Erfindung wird durch die in den Figuren dargestellten Ausführungsbeispiele näher beschrieben:

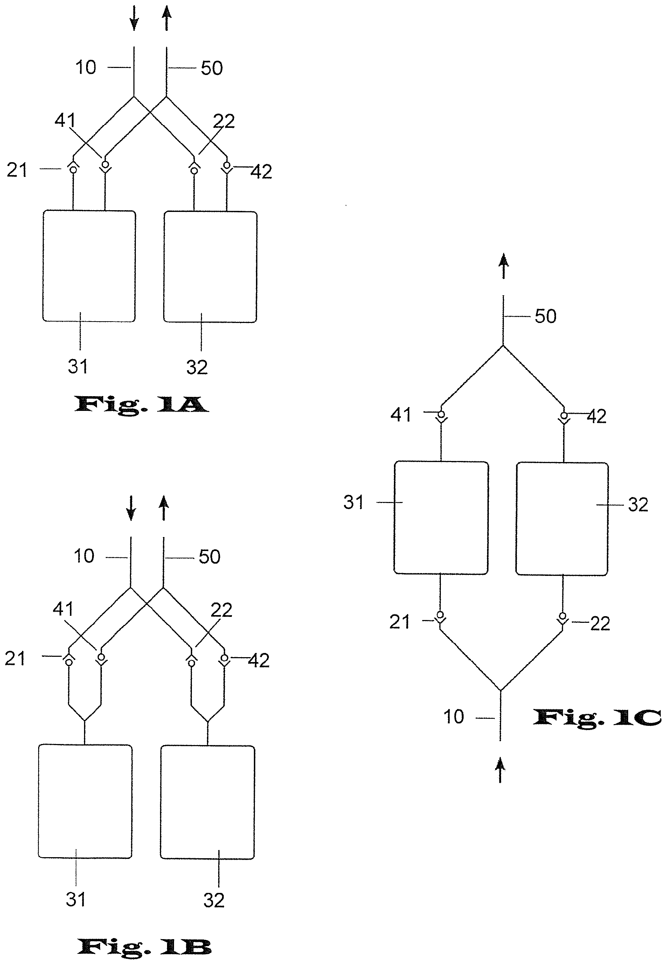

Figur 1A, 1B und 1C zeigen schematisch den Aufbau und die Verschaltung verschiedener bevorzugter Varianten der erfindungsgemäßen Pumpanordnung zur Verwendung beider unidirektionalen insbesondere pulsartilen Perfusion. Die Pumpanordnung enthält einen Einlassport (10), welcher in Fluidverbindung steht mit einem ersten und einem zweiten Einlassventil (21, 22). Erstes und zweites Einlassventil (21, 22) stehen mit den beiden flexiblen Flüssigkeitskammern (31, 32) in Fluidverbindung. Erste und zweite Flüssigkeitskammer (31, 32) ist wiederum mit einem ersten beziehungsweise zweiten Auslassventil (41, 42) verbunden, deren Ausgänge sich in einem Auslassport (50) vereinen. In den in Figuren 1A und 1C dargestellten Varianten weisen die Flüssigkeitskammern (31, 32) jeweils zwei separate Zu- und Ableitungen zu den jeweiligen Einlass- und Auslassventilen (21, 22, 41, 42) auf. In der Variante nach Figur 1B weisen die Flüssigkeitskammern (31, 32) jeweils nur einen gemeinsamen Zu- und Ablauf auf, der sich dann jeweils in Zu- und Ableitungen zu den jeweiligen Einlass- und Auslassventilen (21, 22, 41, 42) aufzweigt.

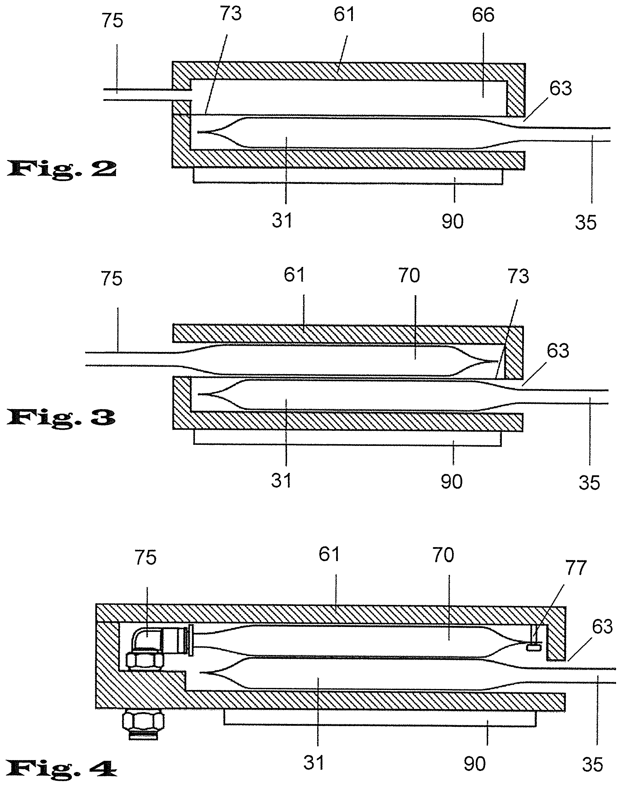

Figur 2 zeigt eine erste Ausgestaltung eines erfindungsgemäßen Pumpenaufbaus, worin in einem starren Gehäuse (61) eine Luftkammer (66) ausgebildet ist, welche über eine Pneumatikleitung (75) insbesondere periodisch druckbeaufschlagt werden kann. Zur Abdichtung der Druckkammer (61) ist eine flexible Membran oder verschiebliche Wand (73) angeordnet. Die Luftkammer (66) fungiert in Verbindung mit einer die Kammer abdichtenden flexiblen und/oder elastischem Membran oder verschieblichen Wand (73) bei Druckbeaufschlagung als pneumatischer Verdrängungsaktor. Er verdrängt eine in das starre Gehäuse (61) eingelegte flexible Flüssigkeitskammer (31) mit Zu- und Ableitung (35), wodurch diese und deren Inhalt druckbeaufschlagt wird. In der dargestellten Ausführung weist das starre Gehäuse (61) eine schlitzförmige Öffnung (63) auf, durch welche die flexible Flüssigkeitskammer (31) in das Gehäuse einlegbar und bevorzugt dort fixierbar ist. In der dargestellten Ausführung weist das starre Gehäuse (61) einen Wärmetauscher (90) auf. Der Wärmetauscher (90) steht in Wärmeflussverbindung mit der flexiblen Flüssigkeitskammer (31).
Figur 3 zeigt eine Variante der Anordnung nach Figur 2. Abweichend von der Anordnung nach Figur 2 ist hier anstelle der fest in dem starren Gehäuse (61) ausgebildeten Luftkammer (66) einen Raum zur Aufnahme eines flexiblen Luftbeutels (70) vorgesehen, der über die Pneumatikleitung (75) periodisch mit Druck beaufschlagt werden kann. Der Luftbeutel (70) wirkt als Verdrängungsaktor. Er steht bei eingelegter flexibler Flüssigkeitskammer (31) bevorzugt über die flexible und/oder elastische Membran oder verschiebliche Wand (73) in mechanischem Kontakt; eine Druckbeaufschlagung des Luftbeutels (70) führt unmittelbar zu einer Druckbeaufschlagung der flexiblen Flüssigkeitskammer (31) und deren Inhalt. In der Ausführung nach Figur 3 ist der Luftbeutel (70) separat und über eine Öffnung aus dem starren Gehäuse (61) herausnehmbar beziehungsweise auswechselbar.
Figur 4 zeigt eine weitere Variante der Anordnungen nach den Figuren 2 und 3. Der Verdrängungsaktor wird durch einen in das starre Gehäuse (61) fest eingebauten Luftsack 70 gebildet, der in dem Gehäuse insbesondere über die Pneumatikleitung (75) und gegebenenfalls zusätzliche Befestigungsmittel (77) in dem Gehäuse fest fixiert ist. Eine in das starre Gehäuse (61) eingesetzte flexible Flüssigkeitskammer (31) kommt mit dem eingebauten Luftbeutel bevorzugt unmittelbar in Kontakt.

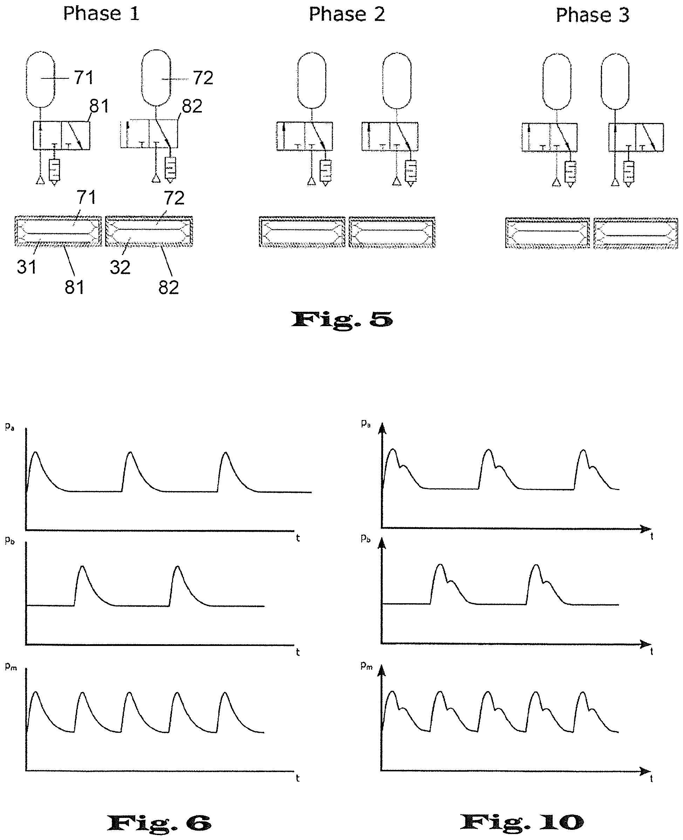

Figur 5 zeigt in einer schematischen Darstellung als Fluidik-Schaltplan drei sequentielle und sich in der Abfolge wiederholende Pumpphasen des Antriebs der beiden pneumatischen Verdrängungsaktoren (71,72) über die zugeordneten Ventile (81,82). Figur 5 zeigt weiter den Schnitt durch die Gehäuseteile (61,62) mit den jeweiligen Verdrängungsaktoren (71,72) und den jeweils darin angeordneten flexiblen Flüssigkeitskammern (31,32) zu jeder der drei Pumpphasen. Phasen 1 und 2 sind zwingend, Phase 3 ist optional, aber bevorzugt in der Abfolge enthalten.
Figur 6 zeigt schematisch den Druckverlauf in den beiden Kammern (31,32) (pa, pb) und im gepumpten Medium (pm) außerhalb der Pumpe.

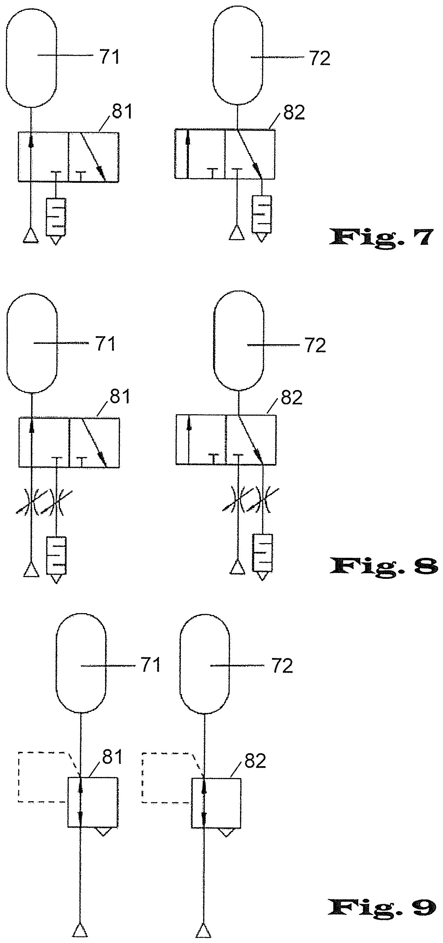

Figur 7 zeigt schematisch einen Schaltplan zur Befüllung und Entlüftung der pneumatischen Verdrängungsktoren (71, 72) bei einer Ausgestaltung mit Wege- oder Sperrventilen (81, 82).
Figur 8 zeigt schematisch einen Schaltplan zur Befüllung und Entlüftung der pneumatischen Verdrängungsktoren (71, 72) bei einer Ausgestaltung mit Wege- oder Sperrventilen (81,82) mit einstellbarer Flankensteilheit.
Figur 9 zeigt schematisch einen Schaltplan zur direkten Regelung des Drucks in der pneumatischen Verdrängungsaktoren bei einer Ausgestaltung mit Proportional-Druckregelventilen (81, 82).
Figur 10 zeigt schematisch den physiologischen Druckverlauf mit charakteristischem Doppel-Peak als Beispiel für eine beliebig modellierbare Druckverlaufskurve in den beiden Beuteln (pa, pb) und im Medium (pm).

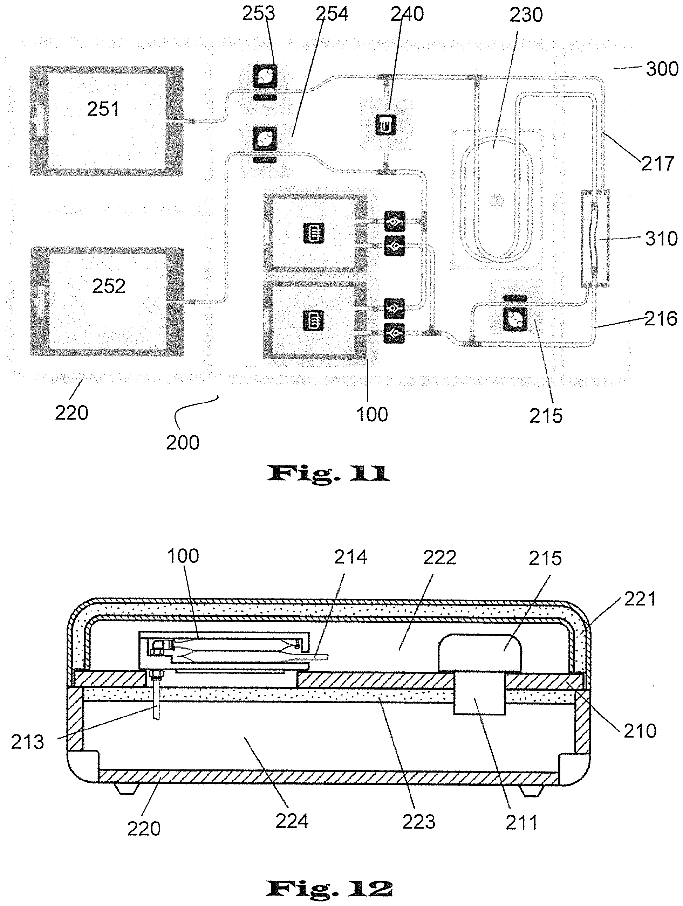

Figur 11 zeigt eine Übersicht über den Aufbau des Flüssigkeitspfads und der funktionellen Komponenten in einem erfindungsgemäßen Bioreaktorsystem. In einem Gehäuse (220) befinden sich die Funktionskomponenten des Hauptmoduls (200): die erfindungsgemäße Pumpanordnung (100), enthaltend die flexiblen Flüssigkeitskammern und die passiven Einlass- und Auslassventile in einem starren Gehäuse mit pneumatischer Druckbeaufschlagung, einen Blasenfänger (240), eine Gasaustauschkammer (230), eine erste Schlauchpumpe (215) für einen Nebenkreislauf (217), Schlauchpumpen (253) und (254) zur Ab- beziehungsweise Zufuhr von Abfall-Medium beziehungsweise Frischmedium, einen Abfalltank (251), einen Vorratstank (252). Die dargestellte Ausführung enthält einen Hauptkreislauf (216), der über die Pumpe (100) gespeist wird, zur Perfusion des Gewebes in der Gewebekammer (310) des separaten Gewebekammermoduls (300) und einen Nebenkreislauf (217) der über die Schlauchpumpe (215) gespeist wird, zur Perifusion des Gewebes in der Gewebekammer (310).
Figur 12 zeigt einen Querschnitt mit Fluidik- und Elektronikkompartiment eines Hauptmoduls (200), enthaltend die Pumpe (100) mit einer Basisplatte (210), einem abnehmbaren Deckel mit thermischer Isolierung (221), Druckluftanschluss (213), Verbindung zu Perfusionskreislauf (214), Kopf einer Schlauchpumpe (215), hier gezeichnet als Beispiel für ein Panel Mount Element, einem Antrieb (211) einer Schlauchpumpe, dem anwenderzugänglichen Fluidik-Kompartiment (222), einer thermischen Isolierung (223) unter der Basisplatte (210) und einem davon getrennten Kompartiment (224) für Elektronik und Pneumatik.

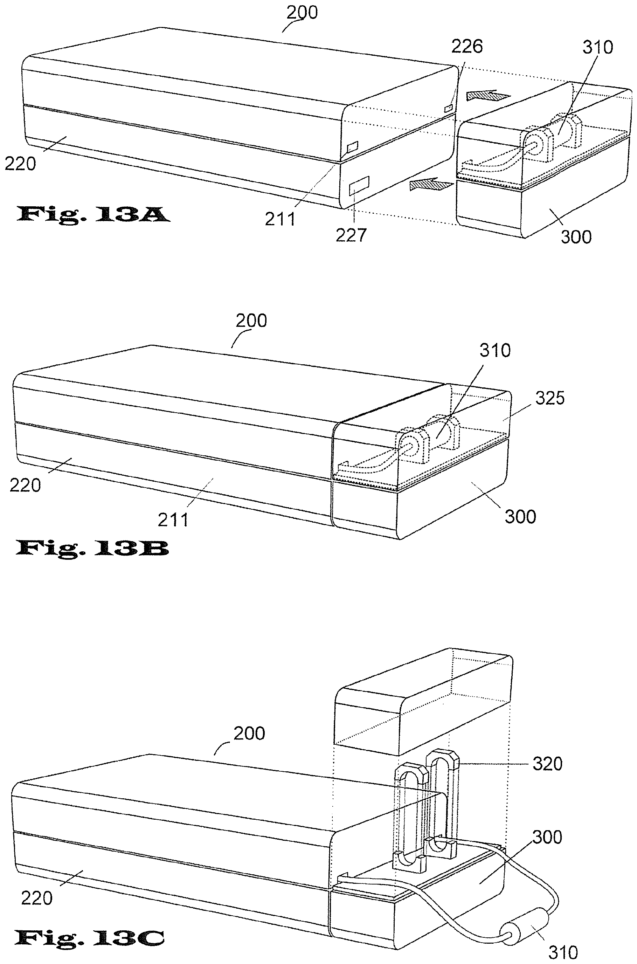

Figuren 13A, 13B, 13C zeigen perspektivische Ansichten des modularen Konzepts aus Hauptmodul (200) und Gewebekammermodul (300) mit Gehäuse (220) für Hauptmodul (200), elektrischer Schnittstelle (222) (Sensorik, Aktorik, Heizung etc.) zum Gewebekammermodul (300), mindestens einer Durchlassöffnung (227) für Schläuche, Fließrichtung in die Gewebekammer (310), mindestens einer Durchlassöffnung (226) für Schläuche, Fließrichtung aus der Gewebekammer (310) sowie Gewebekammermodul (300) mit Gewebekammer (310) und transparenter Haube (325). Figur 13C zeigt eine bei laufendem Betrieb herausgenommene Gewebekammer (310) (z.B. für mikroskopische Untersuchungen) und die Halterung (320) der Gewebekammer (310) in dem Gewebekammermodul (300) im geöffneten Zustand.

### Beispiel 1: Pneumatische Pumpanordnung für pulsatile Perfusion

Die Pumpe besteht aus zwei flüssigkeitsgefüllten Beuteln die abwechselnd pneumatisch komprimiert werden. Die Zuleitung zweigt sich auf und mündet jeweils in einem Beutel während Rückschlagventile dafür sorgen, dass die Flüssigkeit bei der Kompression des Beutels nur in Richtung der Ableitung fließen kann. In der Ableitung für die gepumpte Flüssigkeit jedes Beutels befindet sich ebenfalls jeweils ein Rückschlagventil, das den Rückstrom der Flüssigkeit zurück in den Beutel verhindert. Die Ableitungen der beiden Beutel vereinigen sich nach den Ventilen in einen gemeinsamen Auslass.

Zur Kraftübertragung zwischen Luft und Flüssigkeit, d.h. zur Druckbeaufschlagung der Flüssigkeitsbeutel (31, 32) befinden sich diese jeweils in Verbindung mit jeweils einem Aktor (71, 72) in Form eines aufblasbaren Luftkompartiments (z.B. ein Luftbalg oder eine durch eine flexible Membran getrennte Luftkammer) einem festen Gehäuse oder in einer flexiblen Druckmanschette. Alternativ wird als Aktor ein gleicher Beutel wie für die Flüssigkeit verwendet. Der in dem Luftkompartiment gemessene Druck entspricht genau dem Flüssigkeitsdruck. Der Druck im Flüssigkeitssystem wird nichtinvasiv gemessen und für eine dynamische Drucksteuerung eingesetzt.

Die Pumpwirkung kommt durch das alternierende Komprimieren der Luftbeutel (71, 72) mittels Druckluft zustande. Dabei dient jeweils der drucklose Beutel als Reservoir zur Aufnahme der aus dem Kreislauf zurückströmenden Flüssigkeit. Ein Pumpzyklus kann in drei Phasen unterteilt werden (siehe Fig. 5). In der aktiven Phase 1 wird der erste Luftbeutel (71) mit Druckluft versorgt und der zweite Luftbeutel (72) entlüftet. Das Medium wird dadurch aus dem Flüssigkeitsbeutel (31) herausgedrückt, strömt durch das System und danach in den zweiten Flüssigkeitsbeutel (32) der sich passiv dehnt. In Phase 2 werden beide Luftbeutel (71, 72) belüftet; es füllen sich beide Flüssigkeitsbeutel (31, 32) passiv mit Medium. Die Phase 2 ist optional. In der aktive Phase 3 wird der erste Luftbeutel (71) entlüftet und der zweite Luftbeutel (72) mit Druckluft beaufschlagt. Das Medium wird dadurch aus dem zweiten Flüssigkeitsbeutel (32) herausgedrückt, strömt in das System und danach zurück in den ersten Flüssigkeitsbeutel (31).

Durch die Anordnung der passiven Rückschlagventile (21, 22, 41, 42) an den Flüssigkeitsbeuteln (31, 32) herrscht im System immer der gleiche Druck wie in den mit Druckluft versorgtem Luftbeuteln (71, 72). Der Wechselanteil der entstehenden Druckkurve folgt dem Superpositionsprinzip (siehe Fig. 6).

Für Zellkultur und Tissue Engineering sind die Bereiche, die Zellen enthalten, temperiert. Die Beutel in der Pumpkammer für den Wärmeaustausch vorgesehen. In dem Gehäuse der Pumpe ist als Wärmetauscher eine elektrisch beheizte Platte integriert oder das gesamte Gehäuse, das aus wärmeleitfähigem Material (Aluminium) hergestellt, ist beheizt. Das zu ermöglichte direkte Beheizen der Flüssigkeitsbeutel ermöglicht schnelleren Wärmeübertrag in die Flüssigkeit und damit eine kontrollierbare Temperierung im gesamten System.

Weiter ist ein Windkessel (compliance chamber) zum Druckausgleich und zur Strömungsglättung in das Fluidiksystem eingebaut, und zwar in Flussrichtung nach der Pumpe. Damit lässt sich ein kontinuierlicherer Fluss erzeugen. Die Dimensionierung des Windkessels ist so gestaltet, dass nur die kurze Umschaltphase zwischen der alternierenden Druckbeaufschlagung der Beutel überbrückt oder ausgeglichen wird.

Als Flüssigkeitskammern oder Beutel werden medizinische Infusionsbeutel oder Zellkulturbeutel, insbesondere Infusionsbeutel mit zwei seitlichen Anschlüssen eingesetzt. Das Gehäuse der Pumpe ist so ausgeführt, dass die Beutel einfach von einer Seite durch einen Schlitz in die Kammer eingeschoben sind.

Variante der Ausführung: Die mit Luft gefüllten Beutel können mit Hilfe von Wegeventilen befüllt beziehungsweise geleert werden (siehe Fig. 7). Die Schlagfrequenz kann über die Taktung der Ventile eingestellt werden. Dabei lässt sich der obere Druck im Medium durch den Arbeitsdruck der Pneumatik einstellen. Der untere Druck lässt sich nicht regeln und ist abhängig vom angeschlossenen Fluidsystem. Zur Justage des unteren Drucks und der Flankensteilheiten können Drosselventile eingesetzt werden, die die Geschwindigkeit, mit der der Beutel geleert beziehungsweise gefüllt wird verändern (siehe Fig. 8).

Variante der Ausführung: Die mit Luft gefüllten Beutel können auch mit Hilfe von Wegeventilen befüllt beziehungsweise geleert werden (siehe Fig. 7). Der obere und untere Druck lässt sich einstellen indem zwischen den beiden Kammern umgeschaltet wird, sobald der jeweilige Druck erreicht ist. Dabei ist allerdings die Schlagfrequenz abhängig vom angeschlossenen Fluidsystem. Zur Justage der Schlagfrequenz und der Flankensteilheiten können Drosselventile eingesetzt werden, dass die Geschwindigkeit, mit der der Beutel geleert wird verändert (siehe Fig. 8).

Variante der Ausführung: Die mit Luft gefüllten Beutel können über ein Proportional-Druckregelventil befüllt beziehungsweise geleert werden (siehe Fig. 9). Der Luftdruck lässt sich elektrisch durch das Ventil einstellen. So kann man beliebige Druckverläufe modulieren. Das heißt der obere und untere Druck und die Schlagfrequenz lassen sich unabhängig voneinander und vom angeschlossenen Fluidsystem einstellen.

### Beispiel 2: Skalierbares und Modulares Perfusions-Bioreaktorsvstem

Das aufgebaute modulare Bioreaktorsystem (siehe Fig. 11) besteht zumindest aus den folgenden Komponenten:
1) Hauptmodul mit:
   - Pumpe für die pulsatile oder gleichmäßige Perfusion des Gewebemodells,
   - Pumpe für Perifusion (Umspülung) des Gewebemodells,
   - Gasaustauscher in Form eines gasdurchlässigen Schlauchs, der in einer Kammer verläuft in der eine definierte Atmosphäre eingestellt werden kann,
   - Blasenfänger,
   - Heizung,
   - gekühltes Kompartiment für Frischmedium, und
   - Steuerelektronik und Rechner zur Prozesssteuerung
   und
2) Gewebekammermodul mit:
   - Gewebekammer mit zwei geeigneten Anschlüssen für den Perfusionskreislauf und optional zwei Anschlüssen für den Perifusionskreislauf

Die Funktionskomponenten sind in einem integralen Hauptmodul untergebracht und angeordnet und sind so auf bzw. in einer Grundplatte befestigt, dass von der Anwenderseite das Flüssigkeitssystem eingelegt werden kann, während die Versorgung der Komponenten mit Strom, Druckluft und Messleitungen auf der vom Nutzer abgewandten Seite geschieht (siehe Fig. 12). Die Grundplatte ist in ein Gehäuse eingelassen und trennt dort das Elektronik-Kompartiment vom anwenderzugänglichen Fluidik-Kompartiment. Das Fluidik-Kompartiment ist mit einem thermisch isolierten Deckel schließbar. Statt wie in einem Zellkulturinkubator Luft als Kopplungsmedium zu nutzen wird in unserem Aufbau der Fluidpfad direkt beheizt. Die Auflageflächen für die Beutel in der Beutelpumpe sind aus einem leitfähigen Material gefertigt (z.B. Aluminium). Auf der den Beuteln gegenüberliegenden Seite sing Heizelemente angebracht. Zusammen mit einem in die Aluminiumfläche eingelassenen Temperatursensor wird die Temperatur geregelt. Durch die große Auflagefläche und das dünne Beutelmaterial ergibt sich ein guter Wärmeübertrag. Der pH im Medium wird über die CO2-Konzentration eingestellt (für Bikarbonat-gepufferte Medien). Dazu wird Medium durch einen gasdurchlässigen Schlauch gepumpt der durch eine Kammer läuft in der die gewünschte CO2 Atmosphäre durch Mischung von Luft und CO2 eingestellt wird. Es findet eine automatische Prozessteuerung statt: Medienwechsel erfolgt in einer konstanten Rate oder basierend auf Messung von Nährstoffen und Stoffwechselprodukten (Glukose und Laktat). Vom Hauptkreislauf zweigt ein Nebenkreislauf ab, der von einer eigenen Pumpe versorgt wird. Dieser Nebenkreislauf dient der Perifusion, also Umspülung, des Gewebemodells.

Das Fluidsystem ist geschlossen und kann nach der Sterilisation gelagert werden kann. Das System bleibt geschlossen bis auf die Momente in denen das Medienreservoir angeschlossen bzw. gewechselt wird oder die Zellen in das System injiziert werden. Der Flüssigkeitspfad kann als geschlossenes Kreislaufsystem bestehend aus Beuteln, Schläuchen, Konnektoren, Blasenfänger und Gewebekammer im Ganzen in den Bioreaktor eingelegt werden.

Die Gewebekammer ist im Gewebekammermodul so in einem Halter angebracht, dass es möglich ist, im laufenden Betrieb die Gewebekammer herauszunehmen ohne die Integrität und Sterilität des Flüssigkeitssystems zu stören. Das Flüssigkeitssystem verfügt über ausreichend lange Verbindungsschläuche zur Gewebekammer, dass die Gewebekammer in einem geeigneten externen Untersuchungsgerät (z.B. Mikroskop, Spektrometer, etc.) platziert werden kann. Zum Entnehmen der Gewebekammer muss nur das Gewebekammermodul geöffnet werden wodurch der Wärmeverlust minimiert wird (siehe Fig. 13C).

Durch die Gewebekammer und den eingesetzten Zellträger wird das Fluidsystem anwendungsspezifisch. Die Gewebekammer ist mit einem Barcode markiert, der von einem Scanner im Gewebekammermodulerkannt wird. Das Softwaresystem nutzt diese Information um einen anwendungsspezifischen Satz von Konfigurationsparametern für den Herstellungs- und Kultivierungsprozess zu laden. Das Bioreaktorsystem folgt einem modularen Aufbau, indem der Austausch der Gewebekammer möglich ist (siehe Fig. 13A, 13B). Eine geeignete Schnittstelle sorgt für die Kommunikation zwischen Hauptsteuerung und Gewebekammermodul. Variable anwendungsspezifische Gewebekammermodule haben jeweils eine digitale Signatur, die der Steuersoftware eine automatische Erkennung ermöglicht.

## Patentansprüche

1. Pumpanordnung (100) zur unidirektionalen Perfusion biologischer Zellen und Gewebe, enthaltend:
- eine erste flexible Flüssigkeitskammer (31),
- ein erstes passives Einlassventil (21), das der ersten Flüssigkeitskammer (31) zugeordnet ist und mit dieser in Fluidverbindung steht,
- ein erstes passives Auslassventil (41), das der ersten Flüssigkeitskammer (31) zugeordnet ist und mit dieser in Fluidverbindung steht, und
- eine zweite flexible Flüssigkeitskammer (32),
- ein zweites passives Einlassventil (22), das der zweiten Flüssigkeitskammer (32) zugeordnet ist und mit dieser in Fluidverbindung steht,
- ein zweites passives Auslassventil (42), das der zweiten Flüssigkeitskammer (32) zugeordnet ist und mit dieser in Fluidverbindung steht,
wobei die Eingänge von erstem und zweitem Einlassventil (21,22) gemeinsam mit einem Einlassport (10) in Fluidverbindung stehen und wobei die Ausgänge von erstem und zweitem Auslassventil (41,42) gemeinsam mit einem Auslassport (50) in Fluidverbindung stehen, und weiter enthaltend:
- ein erstes starres Gehäuseteil (61) zur Aufnahme der ersten Flüssigkeitskammer (31), enthaltend einen ersten Verdrängungsaktor (71), zur Druckbeaufschlagung der ersten Flüssigkeitskammer (31), und
- ein zweites starres Gehäuseteil (62) zur Aufnahme der zweiten Flüssigkeitskammer (32), enthaltend einen zweiten Verdrängungsaktor (72), zur Druckbeaufschlagung der zweiten Flüssigkeitskammer (32).

2. Pumpanordnung nach Anspruch 1, wobei der Verdrängungsaktor (71,72) ein flexibler pneumatisch druckbeaufschlagbarer Luftbeutel (70) ist.

3. Pumpanordnung nach Anspruch 1, wobei der Verdrängungsaktor (71,72) eine pneumatisch druckbeaufschlagbare druckdichte Luftkammer in dem Gehäuse (61,62) ist, welche jeweils über eine flexible Membran (73,74) mit der Flüssigkeitskammer (31,32) in Wirkverbindung steht.

4. Pumpanordnung nach einem der vorstehenden Ansprüche, wobei zumindest ein Verdrängungsaktor (71,72) mit mindestens einem Drucksteuerventil (81,82) in Verbindung steht zur wiederkehrenden druckgesteuerten Druckbeaufschlagung des Verdrängungsaktors (71,72)

5. Pumpanordnung nach Anspruch 4, wobei erster und zweiter Verdrängungsaktor (71,72) jeweils mit einem ersten und zweiten aktiven Drucksteuerventil (81,82) in druckfester Verbindung steht zur alternierenden druckgesteuerten Druckbeaufschlagung der Verdrängungsaktoren (71,72).

6. Pumpanordnung nach einem der Ansprüche 4 oder 5, wobei das Drucksteuerventil (81,82) ein Proportional-Druckregelventil ist.

7. Pumpanordnung nach einem der vorstehenden Ansprüche, wobei die Einlassventile (21,22) und Auslassventile (41,42) jeweils über eine gemeinsame Zuleitung (43,44) mit den ihnen zugeordneten Flüssigkeitskammern (31,32) in Fluidverbindung stehen.

8. Pumpanordnung nach einem der vorstehenden Ansprüche, wobei in oder am dem Gehäuseteil (61,62) ein Wärmetauscher (90) unmittelbar integriert ist zur Temperierung der Flüssigkeitskammern (31,32).

9. Pumpanordnung nach einem der vorstehenden Ansprüche, wobei das Gehäuseteil (61,62) einen seitlichen Schlitz (63,64) zur Aufnahme der Flüssigkeitskammer (31,32) aufweist

10. Pumpanordnung nach einem der vorstehenden Ansprüche, wobei die Flüssigkeitskammern (31,32) sterilisierbare Einweg-Folienbeutel sind.

11. Hauptmodul (200) eines Bioreaktors zur Perfusion von biologischen Zellen oder Gewebe in einem damit verbindbaren Gewebekammermodul (300) enthaltend:
- Pumpe (100) nach einem der vorstehenden Ansprüche,
- Fluidikanordnung (120) zur Fluidverbindung der Pumpe (100) und dem verbindbaren Gewebekammermodul (300),
- Drucksteuersystem (130) zur Steuerung der Druckbeaufschlagung des ersten und/oder zweiten Verdrängungsaktors (71,72) der Pumpe (100),
- Temperiereinheit mit Wärmetauscher (90)

12. Hauptmodul nach Anspruch 11, wobei das Drucksteuersystem (130) enthält:
- mindestens ein Drucksteuerventil (81,82), das mit mindestens einem Verdrängungsaktor (71,72) in druckfester Verbindung steht,
- einen Rechner (132), der mit dem Drucksteuerventil (81,82) verbunden ist zu dessen programmgemäßer Ansteuerung.
- mindestens ein mit dem Rechner (132) verbundener Drucksensor (134) zur direkten oder indirekten Messung des aktuellen Drucks in dem Bioreaktorsystem.

13. Modulares Bioreaktorsystem, enthaltend:
- generisches Hauptmodul (200) nach einem der Ansprüche 10 bis 12 und
- anwendungsspezifisches Gewebekammermodul (300) zur Aufnahme von biologischen Zellen oder Gewebe,
wobei das Gewebekammermodul (300) von dem Hauptmodul (200) räumlich getrennt und austauschbar ist.

14. Modulares Bioreaktorsystem nach Anspruch 13, wobei das Gewebekammermodul (300) von dem Hauptmodul (200) im laufenden Kultivierungsbetrieb entfernbar ist.

## Claims

1. Pump arrangement (100) for unidirectional perfusion of biological cells and tissue, containing:
- a first flexible liquid chamber (31),
- a first passive inlet valve (21) which is assigned to and fluidically connected to the first liquid chamber (31),
- a first passive outlet valve (41) which is assigned to and fluidically connected to the first liquid chamber (31), and
- a second flexible liquid chamber (32),
- a second passive inlet valve (22) which is assigned to and fluidically connected to the second liquid chamber (32),
- a second passive outlet valve (42) which is assigned to and fluidically connected to the second liquid chamber (32),
wherein the inputs of the first and second inlet valves (21, 22) are jointly fluidically connected to an inlet port (10), and wherein the outputs of the first and second outlet valves (41, 42) are jointly fluidically connected to an outlet port (50), and further containing:
- a first rigid housing part (61) for receiving the first liquid chamber (31), containing a first displacement actuator (71) for applying pressure to the first liquid chamber (31), and
- a second rigid housing part (62) for receiving the second liquid chamber (32), containing a second displacement actuator (72) for applying pressure to the second liquid chamber (32).

2. Pump arrangement according to claim 1, wherein the displacement actuator (71, 72) is a flexible air bag (70) to which pressure can be applied pneumatically.

3. Pump arrangement according to claim 1, wherein the displacement actuator (71, 72) is a pressure-tight air chamber, to which pressure can be applied pneumatically and which is operatively connected via a flexible membrane (73, 74) to the liquid chamber (31, 32) respectively, in the housing (61, 62).

4. Pump arrangement according to any of the preceding claims, wherein at least one displacement actuator (71, 72) is connected to at least one pressure control valve (81, 82) for recurrent pressure-controlled pressure application from the displacement actuator (71, 72).

5. Pump arrangement according to claim 4, wherein the first and second displacement actuators (71, 72) are in a pressure-tight connection with a first and second active pressure control valve (81, 82) respectively for alternating pressure-controlled pressure application from the displacement actuators (71, 72).

6. Pump arrangement according to either claim 4 or claim 5, wherein the pressure control valve (81, 82) is a proportional pressure regulation valve.

7. Pump arrangement according to any of the preceding claims, wherein the inlet valves (21, 22) and outlet valves (41, 42) are respectively fluidically connected to the liquid chambers (31, 32) assigned thereto via a shared feed line (43, 44).

8. Pump arrangement according to any of the preceding claims, wherein for temperature control of the liquid chambers (31, 32) a heat exchanger (90) is directly integrated into or onto the housing part (61, 62).

9. Pump arrangement according to any of the preceding claims, wherein the housing part (61, 62) has a lateral slot (63, 64) for receiving the liquid chamber (31, 32).

10. Pump arrangement according to any of the preceding claims, wherein the liquid chambers (31, 32) are sterilisable single-use film bags.

11. Primary module (200) of a bioreactor for perfusion of biological cells or tissue in a tissue chamber module (300) connected thereto, containing:
- a pump (100) according to any of the preceding claims,
- a fluidic arrangement (120) for fluidically connecting the pump (100) and the connectable tissue chamber module (300),
- a pressure control system (130) for controlling the pressure application from the first and/or second displacement actuators (71, 72) of the pump (100),
- a temperature-control unit comprising a heat exchanger (90).

12. Primary module according to claim 11, wherein the pressure control system (130) contains:
- at least one pressure control valve (81, 82) which is in a pressure-tight connection with at least one displacement actuator (71, 72),
- a computer (132) which is connected to the pressure control valve (81, 82) to actuate it in accordance with a program,
- at least one pressure sensor (134) connected to the computer (132) for directly or indirectly measuring the current pressure in the bioreactor system.

13. Modular bioreactor system, containing:
- a generic primary module (200) according to any of claims 10 to 12 and
- an application-specific tissue chamber module (300) for receiving biological cells or tissue,
wherein the tissue chamber module (300) is spatially separated from the primary module (200) and is replaceable.

14. Modular bioreactor system according to claim 13, wherein the tissue chamber module (300) is removable from the primary module (200) during continuous cultivation operation.

## Revendications

1. Ensemble de pompage (100) pour la perfusion unidirectionnelle de cellules et de tissus biologiques, comprenant :
- une première chambre de fluide flexible (31),
- une première soupape d'entrée passive (21) qui est associée à la première chambre de fluide (31) et en liaison fluidique avec celle-ci,
- une première soupape de sortie passive (41) qui est associée à la première chambre de fluide (31) et en liaison fluidique avec celle-ci ; et
- une deuxième chambre de fluide flexible (32),
- une deuxième soupape d'entrée passive (22) qui est associée à la deuxième chambre de fluide (32) et en liaison fluidique avec celle-ci,
- une deuxième soupape de sortie passive (42) qui est associée à la deuxième chambre de fluide (32) et en liaison fluidique avec celle-ci,
dans lequel les entrées des première et deuxième soupapes d'entrée (21, 22) sont conjointement en liaison fluidique avec un orifice d'entrée (10) et dans lequel les sorties des première et deuxième soupapes de sortie (41, 42) sont conjointement en liaison fluidique avec un orifice de sortie (50), et comprenant en outre :
- une première partie de boîtier rigide (61) destinée à recevoir la première chambre de fluide (31), comprenant un premier actionneur de déplacement (71) pour mettre sous pression la première chambre de fluide (31) ; et
- une deuxième partie de boîtier rigide (62) destinée à recevoir la deuxième chambre de fluide (32), comprenant un deuxième actionneur de déplacement (72) pour mettre sous pression la deuxième chambre de fluide (32).

2. Ensemble de pompage selon la revendication 1, dans lequel l'actionneur de déplacement (71, 72) est une poche à air flexible (70) pouvant être mise sous pression pneumatiquement.

3. Ensemble de pompage selon la revendication 1, dans lequel l'actionneur de déplacement (71, 72) est une chambre à air étanche à la pression pouvant être mise sous pression pneumatiquement dans le boîtier (61, 62), laquelle est en liaison fonctionnelle avec la chambre de fluide (31, 32) par l'intermédiaire d'une membrane flexible respective (73, 74).

4. Ensemble de pompage selon l'une des revendications précédentes, dans lequel au moins un actionneur de déplacement (71, 72) est en liaison avec au moins une soupape de commande de pression (81, 82) pour la mise sous pression récurrente, commandée par la pression, de l'actionneur de déplacement (71, 72).

5. Ensemble de pompage selon la revendication 4, dans lequel un premier et un deuxième actionneur de déplacement (71, 72) sont chacun en liaison résistante à la pression avec une première et une deuxième soupape de commande de pression active (81, 82) pour la mise sous pression alternée, commandée par la pression, des actionneurs de déplacement (71, 72).

6. Ensemble de pompage selon l'une des revendications 4 ou 5, dans lequel la soupape de commande de pression (81, 82) est une soupape de régulation de pression proportionnelle.

7. Ensemble de pompage selon l'une des revendications précédentes, dans lequel les soupapes d'entrée (21, 22) et les soupapes de sortie (41, 42) sont chacune en liaison fluidique avec les chambres de fluide (31, 32) qui leur sont associées par l'intermédiaire d'une conduite d'amenée commune (43, 44).

8. Ensemble de pompage selon l'une des revendications précédentes, dans lequel un échangeur de chaleur (90) est intégré directement dans ou sur la partie de boîtier (61, 62) pour réguler la température des chambres de fluide (31, 32).

9. Ensemble de pompage selon l'une des revendications précédentes, dans lequel la partie de boîtier (61, 62) présente une fente latérale (63, 64) destinée à recevoir la chambre de fluide (31, 32).

10. Ensemble de pompage selon l'une des revendications précédentes, dans lequel les chambres de fluide (31, 32) sont des poches en film jetables stérilisables.

11. Module principal (200) d'un bioréacteur pour la perfusion de cellules ou de tissus biologiques dans un module de chambre tissulaire (300) pouvant y être relié, comprenant :
- une pompe (100) selon l'une des revendications précédentes,
- un ensemble fluidique (120) pour la liaison fluidique de la pompe (100) et du module de chambre tissulaire (300) pouvant être relié,
- un système de commande de pression (130) pour commander la mise sous pression du premier et/ou du deuxième actionneur de déplacement (71, 72) de la pompe (100),
- une unité de régulation de température avec un échangeur de chaleur (90).

12. Module principal selon la revendication 11, dans lequel le système de commande de pression (130) comprend :
- au moins une soupape de commande de pression (81, 82) en liaison résistante à la pression avec au moins un actionneur de déplacement (71, 72),
- un ordinateur (132) qui est relié à la soupape de commande de pression (81, 82) pour sa commande selon un programme,
- au moins un capteur de pression (134) relié à l'ordinateur (132) pour mesurer directement ou indirectement la pression actuelle dans le système de bioréacteur.

13. Système de bioréacteur modulaire, comprenant :
- un module principal générique (200) selon l'une des revendications 10 à 12 ; et
- un module de chambre tissulaire (300) spécifique à l'application destiné à recevoir des cellules ou des tissus biologiques,
dans lequel le module de chambre tissulaire (300) est séparé spatialement du module principal (200) et échangeable.

14. Système de bioréacteur modulaire selon la revendication 13, dans lequel le module de chambre tissulaire (300) peut être retiré du module principal (200) pendant que l'activité de culture est en cours.
